# EUROPÄISCHE PATENTANMELDUNG

(11) **EP 1 457 489 A1**
(43) Veröffentlichungstag der Anmeldung: **15.09.2004**
(21) Anmeldenummer: 04003352.4
(22) Anmeldetag: 14.02.2004
(51) Int. Cl.: C07D 239/26

(54) **Verfahren zur Herstellung von 4-Alkylpyrimidin**

(30) Priorität: 17.02.2003 DE 10306445
(71) Anmelder: Degussa AG, 40474 Düsseldorf (DE)
(72) Erfinder: Henniges, Hans Dr., 60316 Frankfurt (DE); Pitz, Hans-Jürgen, 51503 Rösrath (DE); Theis, Christoph Dr., 53859 Niederkassel (DE); Neumann, Manfred Dr., 45770 Marl (DE)

(57) **Zusammenfassung**

Das vorliegende Verfahren beschreibt die Synthese von 4-Alkylpyrimidinen, insbesondere 4-Methylpyrimidin. Das Verfahren läßt sich gut im technischen Maßstab anwenden und kommt ohne den Zusatz von Wasser oder Ammoniumchlorid als Katalysator aus und erlaubt die Herstellung der gewünschten Verbindungen in guten Ausbeuten und ausgezeichneten Reinheiten ohne zeitaufwendige Extraktionsschritte.

Pyrimidine dienen als Intermediate zur Synthese von bioaktiven Wirkstoffen.

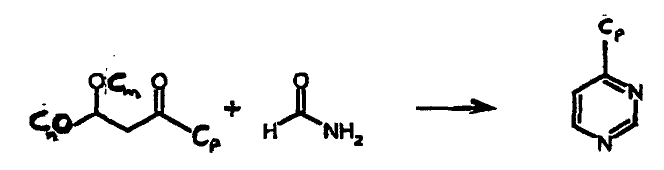

## Beschreibung

Die vorliegende Erfindung bezieht sich auf ein Verfahren zur Herstellung von 4-Alkylpyrimidinen, insbesondere 4-Methylpyrimidin.

4-Methylpyrimidin ist ein Intermediat für die Synthese biologisch aktiver Wirkstoffe wie z.B. Pflanzenschutzmittel oder Pharmaka. Bredereck et al. entwickelten ausgehend von 1,3-Dicarbonylverbindungen und Formamidin Synthesen zum Aufbau von 1,3-Diazolen (Angew. Chem. 1956, 68, 151; Chem. Ber. 1957, 90, 942). Eine Synthese nach Bredereck et al. (Org. Synth. Coll. Vol. V, 794, Wiley 1973) zufolge läßt sich Formamid mit 4,4-Dimethoxy-2-butanon (Formylacetondimethylacetal) in Gegenwart von Ammoniumchlorid und Wasser bei 180-190°C zu 4-

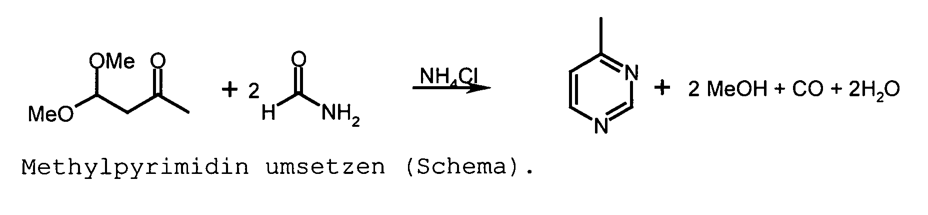

Die dargestellte Methode hat einige Nachteile. Zum einen ist die Ausbeute mit 54-63% für technische Maßstäbe bescheiden. Zum anderen muß man, um die Ausbeute überhaupt zu erreichen, einen Überschuß an Formamid von 6:1 bezogen auf 4,4-Dimethoxy-2-butanon einsetzen, obwohl nur 2 Mol benötigt werden. Weiterhin ist die Zugabe eines leicht sauren Katalysators in 30 Mol% bezogen auf 4,4-Dimethoxy-2-butanon notwendig. Während der anschließenden Destillation sublimieren Ammoniumsalze in den Gasraum und scheiden sich an kühleren Stelle der Reaktionsapparatur ab, was im technischen Maßstab sehr hinderlich ist, können doch solche Salze z.B. Reaktorkühler zusetzen und zu Produktionsausfälle führen.Die nach Bredereck übliche Aufarbeitung des Destillates bedient sich einer langwierigen Extraktion mit Chloroform, um das 4-Methylpyrimidin vom Reaktionswasser und Methanol zu separieren. Dieser ineffiziente Vorgang wird durch die sehr gute Wasserlöslichkeit von 4-Methylpyrimidin bedingt.

Andere Herstellungsverfahren für Pyrimidine gehen von Formamidinium-Salzen aus, was den prinzipiellen Nachteil in sich birgt, daß Formamid meist durch Basenzusatz, üblicherweise ein Alkalialkoholat (z. B. Natriummethylat), freigesetzt werden muß (z.B. JP 08198858, JP 49124081). Auch bei diesen Verfahrensvarianten sind die erzielbaren Ausbeuten meist unbefriedigend. So wird in einem erst vor kurzem veröffentlichten Patent eine Methode verwendet, bei der Formamidiniumacetat eingesetzt wird und anschließend mit tert-Butylmethylether insgesamt 20-26 h extrahiert wird. Das so erhältliche Rohprodukt konnte auf destillativem Wege nur schlecht gereinigt werden. Typisch waren Ausbeuten von 34 bis 50% mit Gehalten von 95-98%. Daneben erhielt man Mischfraktionen mit Gehalten von 88 bis 94%, die wiederum weitere ca. 10% zur Gesamtausbeute beitrugen (DE 10002835).

Aufgabe der vorliegenden Erfindung war deshalb die Angabe eines gegenüber dem Stand der Technik verbesserten Verfahrens zur Herstellung von 4-Alkylpyrimidinen, welches insbesondere eine größere Ausbeute an Produkt zu generieren gestattet und die apparativen Nachteile des Bredereckschen Verfahrens vermeiden hilft. Gerade im technischen Maßstab sollte dieses Verfahren dazu gereichen, 4-Alkylpyrimidine in ökonomisch und ökologisch vorteilhafter Manier herstellen zu können.

Die Aufgabe wird gemäß den Merkmalen des kennzeichnenden Teils von Anspruch 1 gelöst. Ansprüche 2 bis 6 sind auf spezielle Ausführungsformen des erfindungsgemäßen Verfahrens gerichtet.

Dadurch, daß man bei einem Verfahren zur Herstellung von 4-Alkylpyrimidinen ausgehend von Formylmethylalkylketondiacetalen mit Formamid das Formamid vorlegt und dies mit Formylmethylalkylketondiacetalen ohne Zusatz von Wasser bei Temperaturen umsetzt, bei denen das 4-Alkylpyrimidin destillativ aus der Reaktionsmischung entfernt werden kann, erhält man in überraschend hohen dafür aber nicht minder vorteilhaften Ausbeuten die gewünschten Produkte.

Das Formylmethylalkylketondiacetal kann in jeder dem Fachmann für diesen Zweck zur Verfügung stehenden Weise in die Reaktionsmischung dosiert werden. Es kann in toto gleich am Anfang oder vorteilhafterweise kontinuierlich in dem Maße zur Reaktionsmischung hinzu gegeben werden, in dem es durch die Reaktion verbraucht wird.

Die gegenständliche Reaktion ist mit geringen naheliegenden Abweichungen auf alle 4-Alkylpyrimidine anzuwenden. Unter Alkyl wird vorteilhafterweise ein (C₁-C₁₅)-Alkylrest verstanden, besonders vorteilhaft ein Methylrest. In diesem Fall wird als Formylmethylalkylketondiacetalen das 4,4-Dimethoxy-2-butanon (Formylacetondimethylacetal) eingesetzt.

Die Temperatur der Reaktion richtet sich prinzipiell nach der Produktbildung und Isolierung. Die Umsetzung wird vorteilhafterweise bei Temperaturen von >150°C, vorzugsweise >170°C und besonders bevorzugt bei ca. 190°C durchgeführt.

Das generierte Produktgemisch läßt sich nach den Methoden des Standes der Technik (Extraktion, Chromatographie, Kristallisation etc.) aufarbeiten. Besonders bevorzugt kann das Produkt durch fraktionierte Destillation aufgereinigt werden.

Das entstehende Reaktionswasser behindert die effektive Produktbildung dadurch, daß beide zusammen ein azeotropes Gemisch bilden. Um wasserfreies Produkt zu erhalten, muß das Reaktionswasser deshalb aus der Reaktionsmischung entfernt werden. Dies kann nach allen dem Fachmann hierfür zur Verfügung stehenden Mitteln erfolgen wie z.B. Pervaporation durch eine semipermeable Membran, durch Binden des Wassers mit Molsieb oder vorteilhafterweise dadurch, daß man vor der Aufreinigung zur Produktmischung ein wasserbindendes Mittel hinzu gibt. Dies können alle dem Fachmann für diesen Zweck in Frage kommenden Substanzen sein wie z.B. KOH, P₂O₅, PCl₃ oder Orthoester etc. Ganz besonders bevorzugt ist der Zusatz von Trimethylorthoformiat.

Bevorzugt geht man bei dem erfindungsgemäßen Verfahren zur Herstellung von 4-Methylpyrimidin wie folgt vor. Formamid wird in einem Reaktionsgefäß zu 4 Äquivalente vorgelegt und ohne Zusatz von Wasser mit Ammoniumchlorid auf 190°C erhitzt. Sodann dosiert man Formylmethylalkylketondiacetal hinzu. Bei der hohen Temperatur wird, gesteuert durch die Zugabe von Formylmethylalkylketondiacetal, ständig ein Methanol/Wasser/4-Alkylpyrimidin/Formamid-Gemisch abdestilliert. Dadurch wird das Produkt sofort den hohen Reaktionstemperaturen und damit möglichen Nebenreaktionen entzogen. Der Umsatz ist praktisch vollständig. Die Selektivität der Pyrimidinbildung bei der Reaktion liegt häufig über 80% (per GC im Destillat bestimmt). Ammoniumsalze bilden sich sehr viel weniger. Trotzdem sollte die Reaktionsapparatur komplett bei >50°C gefahren werden, um ein Kondensieren von Ammoniumsalzen zu verhindern. Das erhaltene Destillat besteht im wesentlichen aus Ammoniumformiat/Methanol/Wasser/4-Methylpyrimidin/Formamid. Durch einfaches Erwärmen des Destillates auf 60°C kann das Ammoniumformiat ausgetrieben werden. Der Rückstand wird mit soviel Trimethylorthoformiat versetzt, daß alles Reaktionswasser gebunden wird.

Anschließend werden Methylformiat und Methanol abdestilliert, bevor das 4-Alkylpyrimidin übergeht.

Die gegenständliche Erfindung erlaubt die Synthese der kostbaren Intermediate ohne große apparative Schwierigkeiten in einer gegenüber dem Stand der Technik wesentlich vereinfachten und deshalb vorteilhaften Art und Weise. Die Ausbeuten an Produkt können über alles gerechnet bei ca. 80% Reinstsubstanz und höher betragen.

Als Alkylrest wird vorzugsweise ein linearer oder verzweigter, ggf. einfach oder mehrfach ungesättigter (C₁-C₁₅)-Alkylrest verstanden, wie z.B. Methyl, Ethyl, Propyl, Isopropyl, Butyl, tert.-Butyl, sec-Butyl, Isobutyl, Pentyl, Hexyl, Dodecyl- etc.

### Beispiel:

### Reaktion von Formamid und 4,4-Dimethoxy-2-butanon:

Im trockenen Doppelmantel-Glasreaktor wurden 272 g ( 6.0 mol) Formamid vorgelegt und bei guter Rührung auf 190 °C (Sumpftemperatur) erhitzt. Innerhalb von 3 h wurden dann 209 g (1,5 mol) 95%iges 4,4-Dimethoxy-2-butanon mit Hilfe einer Schlauchpumpe kontinuierlich zudosiert. Gleichzeitig wurden über Kopf über ein einfaches, ummanteltes Steigrohr und einen absteigenden, auf 50 °C temperierten Kühler, 342 g eines gelben Reaktionsdestillates abgenommen, das aus Methanol, Wasser, 4-Methylpyrimidin und Ammoniumformiat bestand. Die Kopftemperatur fiel fast unmittelbar nach Ende der Dosierung von ca. 158 °C deutlich ab. Anschließend ließ man noch 30 min nachreagieren.

Das bei der Reaktion als Nebenprodukt gebildete Ammoniumformiat (ca. 15 g) neigt dazu, sich im Gasraum niederzuschlagen bzw. auf der Kühlseite bei zu starker Kühlung auszufallen. Bei der genannten 4,4-Dimethoxy-2-butanon-Dosierrate spülte der Destillatstrom das Salz in die Vorlage.

Die Ölbadtemperaturen lagen bei der Reaktion bei 215-220 °C, Sumpftemperaturen bei 188-196 °C, die Kopftemperaturen stiegen bis auf ca. 158 °C an.

### Salzabtrennung:

Bevor das Reaktionswasser entfernt wird, ist unbedingt das (zum Teil ausgefallene) Ammoniumsalz zu entfernen, da dieses ansonsten während der Zielprodukt-Destillation in den Gasraum sublimiert und sich am Ende mit Gehalten von 2-3% im Produkt-Destillat wiederfindet.

Der Kolben mit dem Reaktionsdestillat (342 g) wurde im Labor mit einem auf 125 °C beheizten Kühler versehen und bei Sumpftemperaturen bis 60 °C (Ölbad bis 68 °C) und Kopftemperaturen bis 39 °C von ca. 15 g Ammoniumsalz befreit. Zeitdauer 1,5 h. Der Rückstand aus 4-Methylpyrimidin, Wasser und Methanol nach der Salzabtrennung betrug 327 g. Die 4-Methylpyrimidin-Gehalte lagen typischerweise bei 34-36 Massen%. Der Wasseranteil bei ca. 12%.

### Wasserabtrennung und Destillation:

Um das Wasser aus dem Gemisch von 4-Methylpyrimidin (ca. 34%), Wasser (ca. 12%) und Methanol zu entfernen, wurde zum Rückstand der Salzabtrennung (327 g) eine Menge von 250 g Trimethylorthoformiat (TMOF) (2.35 mol) gegeben und das Gemisch 30 min bei RT gerührt.

Die anschließende LeichtsiederDestillation erfolgte bei Normaldruck (Abnahme 5 : 1) an einer 60 cm-Füllkörperkolonne (mit Moltifill-Füllkörpern, 10 theor. Böden). Die Ölbadtemperatur wurde hierbei von 89 auf 160 °C erhöht. Die Sumpftemperatur stieg dabei von 69 auf 126 °C und die Kopftemperatur von 34 auf 65 °C, um dann wieder abzufallen (Fraktion 1, ca. 340 g). Die Zielprodukt-Destillation wurde bei 80 mbar an derselben Kolonne durchgeführt. Im Vorlauf wurde die Ölbadtemperatur von 96 auf ca. 120 °C hochgefahren, wobei die Sumpftemperatur von 66 auf 81 °C stieg. Die Kopftemperatur stieg dabei von 31 auf 69 °C an (Fraktion 2, ZP-Vorlauf, ca. 16 g).

Im Hauptlauf (Ölbadtemp. 122-162 °C, Sumpftemp. 80-128 °C, Kopftemp. 69-72 °C) erhielt man bei Einsatz von Vor- und Nachläufen (ca. 35 g) eines anderen, gleichartig gefahrenen Ansatzes ca. 111 g Zielprodukt mit einer Reinheit nach GC von > 99%. Dies entspricht einer Ausbeute von 79% an 4-Methylpyrimidin (Fraktion 3, ZP-Hauptlauf).

## Patentansprüche

1. Verfahren zur Herstellung von 4-Alkylpyrimidinen ausgehend von Formylmethylalkylketondiacetalen mit Formamid,
**dadurch gekennzeichnet, daß**
man Formamid vorlegt und dies mit Formylmethylalkylketondiacetalen ohne Zusatz von Wasser bei Temperaturen umsetzt, bei denen das 4-Alkylpyrimidin destillativ aus der Reaktionsmischung entfernt werden kann.

2. Verfahren nach Anspruch 1,
**dadurch gekennzeichnet, daß**
man das Formylmethylalkylketondiacetal kontinuierlich in dem Maße zur Reaktionsmischung hinzu gibt, in dem es durch die Reaktion verbraucht wird.

3. Verfahren nach Anspruch 1 und/oder 2,
**dadurch gekennzeichnet, daß**
man Formylacetondimethylacetal einsetzt.

4. Verfahren nach einem oder mehreren der vorhergehenden Ansprüche,
**dadurch gekennzeichnet, daß**
man die Umsetzung bei Temperaturen von >150°C, vorzugsweise >170°C und besonders bevorzugt bei ca. 190°C durchführt.

5. Verfahren nach einem oder mehreren der vorhergehenden Ansprüche,
**dadurch gekennzeichnet, daß**
das Produkt durch fraktionierte Destillation aufgereinigt wird.

6. Verfahren nach Anspruch 5,
**dadurch gekennzeichnet, daß**
man vor der Aufreinigung zur Produktmischung ein wasserbindendes Mittel gibt.
